# EUROPEAN PATENT SPECIFICATION

(11) **EP 3 946 188 B1**
(45) Date of publication and mention of the grant of the patent: **07.05.2025**
(21) Application number: 20717057.2
(22) Date of filing: 13.03.2020
(51) Int. Cl.: A61F 13/08, D04B 1/00

(54) **KNITTED ANTI-SLIP ARTICLE**
GEWIRKTER RUTSCHHEMMENDER GEGENSTAND
ARTICLE ANTIGLISSANT TRICOTÉ

(30) Priority: 29.03.2019 US 201962826364 P; 12.03.2020 US 202016817126
(43) Date of publication of application: 09.02.2022
(73) Proprietor: Elastic Therapy, LLC, Asheboro, North Carolina 27205 (US)
(72) Inventor: LINEBERRY, Donnie Hampton, Asheboro, North Carolina 27205 (US); SMITH, Gregory Ted, Asheboro, North Carolina 27205 (US)
(74) Representative: Grünecker Patent- und Rechtsanwälte PartG mbB
(86) International application number: PCT/US2020/022709
(87) International publication number: WO 2020/205208

(56) References cited:
- WO-A1-2011/116952
- WO-A1-2014/098928
- WO-A1-2015/094385
- US-A1- 2003 213 269

## Description

### BACKGROUND

### Field

This disclosure generally relates to an anti-slip portion of a knitted product. More particularly, this disclosure relates to a knitted anti-slip fabric portion of a device or garment that may be used, for example, in compression hosiery or a knee brace. From WO 2014/098928 A1, a therapeutic compression garment with structural features on the inner surface to contact the skin of the wearer is known.

### SUMMARY

The invention is defined by the claims.

Disclosed herein is a knitted orthopedic medical device comprising a knitted article including a knitted anti-slip portion, the anti-slip portion having a knitted surface including a repeating pattern of yarns. In an embodiment, the repeating pattern of yams can include a first low friction yam, a high friction yarn, and a second low friction yarn. In an example, the first low friction yarn can be a 1/70/34 S twist nylon yam, the first high friction yarn can be a 200 dtex silicone yarn, and the second low friction yarn can be a 1/70/34 Z twist nylon yarn. In another embodiment, the repeating pattern of yarns can include a first low friction yarn, a first high friction yarn, a second low friction yam and a second high friction yarn. In an example, the first low friction yarn can be a 1/70/34 S twist nylon yarn, the first high friction yarn can be a 200 dtex silicone yarn, the second low friction yarn can be a 1/70/34 Z twist nylon yarn, and the second high friction yarn comprising 200 dtex silicone yarn. The high friction yarns are held in position with the first and second low friction yarns. The high friction yarn is exposed to contact the wearer's skin in the anti-slip portion so as to increase the anti-slip properties of the garment.

Embodiments of such articles may have one or more additional aspects (or features). In an embodiment, the repeating pattern of the yarns is a jersey stitch. In an embodiment, the repeating pattern is a tuck stitch. In an embodiment, the repeating pattern is a float stitch. Other repeating patterns may also be used. In an example of an article that the anti-slip portion can be knitted into, the knitted article is compression hosiery. The compression hosiery can include a proximal end and a distal end and is shaped to closely fit on a portion of the leg of the wearer of the compression hosiery such that the proximal end is higher on the leg than the distal end, where the knitted anti-slip portion is knitted as a portion of the proximal end. The anti-slip portion can be knitted into the article at an end of the article, e.g., the inside of a welt. In one example, the anti-slip portion on the compression hosiery can be knitted into the article to contact a portion of the leg of a wearer of the compression hosiery between the knee and the ankle. In another example, the anti-slip portion on the compression hosiery can be knitted into the article to contact a portion of the user's leg between the knee and the hip. A knee brace is another example of a knitted article that can incorporate the knitted anti-slip portion. In an embodiment, the knee brace can include a proximal end and a distal end, wherein the knee brace is configured to cover a portion of the leg of a wearer of the knee brace such that the proximal end is higher on the leg than the distal end, where the knitted anti-slip portion is arranged on a portion of the proximal end. In an embodiment, the knee brace can include a proximal end and a distal end, where the knee brace is configured to cover a portion of the leg of a wearer such that the proximal end is higher on the leg than the distal end, where the knitted anti-slip portion is arranged on a portion of the proximal end and the distal end. An elbow brace is another example of an article that can incorporate the anti-slip portion. Such articles include a knitted portion (e.g., the body of the article) that is not part of the knitted anti-slip portion. The body of the article and the knitted anti-slip portion are both formed in the article on the same knitting machine.

Disclosed herein is also a method of knitting a therapeutic medical device that includes a knitted article and a knitted anti-slip portion. The method can include knitting, using a knitting machine, at least a section of an article, the section of the article not having an anti-slip portion, and on the knitting machine without removing the article, knitting an anti-slip portion of the article, the anti-slip portion having yarns arranged in a repeating pattern, yarns can include a first low friction yarn comprising a 1/70/34 S twist S twist yarn, a first high friction yarn comprising 200 dtex silicone yarn, a second low friction yarn comprising a 1/70/34 Z twist yarn, and a second high friction yarn comprising 200 dtex silicone yarn.

Embodiments of such methods may have one or more additional processes or aspects. In various embodiments of the method, the repeating pattern of the yarns can be a jersey stitch, a tuck stitch, or a float stitch. Other repeating patterns may also be used. In an example of an article that the anti-slip portion can be knitted into using such methods, the knitted article is compression hosiery. In another example, the knitted article is a knee brace or an elbow brace. Such articles include a knitted portion (e.g., the body of the article) that is not part of the knitted anti-slip portion. The body of the article and the knitted anti-slip portion are both formed in the article on the same knitting machine.

Disclosed herein is also a knitted orthopedic article having a first knitted section and a second knitted section continuously knit from the first knitted section, the second knitted section having an anti-slip portion including a repeating pattern of yarns. In an embodiment, the repeating pattern of yarns only includes a first low friction yarn comprising a 1/70/34 S twist yarn, a first high friction yarn comprising 200 dtex silicone yarn, a second low friction yarn comprising a 1/70/34 Z twist yarn, and a second high friction yarn comprising 200 dtex silicone yarn, the high friction yarns exposed in the repeating pattern to contact the skin of a wearer of the article so as to increase the anti-slip properties of the garment. In another embodiment, the repeating pattern of yarns only includes a first low friction yarn comprising a 1/70/34 S twist yarn, a high friction yarn comprising 200 dtex silicone yarn, and a second low friction yarn comprising a 1/70/34 Z twist yarn, the high friction yarns exposed in the repeating pattern to contact the skin of a wearer of the article so as to increase the anti-slip properties of the garment.

### BRIEF DESCRIPTION OF THE DRAWINGS

The disclosed aspects will hereinafter be described in conjunction with the appended drawings and appendices, provided to illustrate and not to limit the disclosed aspects, wherein like designations denote like elements.
Figure 1 illustrates an example of a compression garment including an anti-slip portion, according to one embodiment of the invention.
Figure 2 illustrates a portion of the inside surface of the welt of the garment shown in Figure 1.
Figure 3 illustrates a cross-section of the garment of Figure 2.
Figure 4 illustrates a three yarn jersey knit stitch relating to an embodiment of the knitted anti-slip portion, the three yarn jersey knit stitch being repeatable for the entire anti-slip portion.
Figure 5 illustrates a four yarn tuck knit stitch relating to an embodiment of the knitted anti-slip portion, the four yarn tuck knit stitch being repeatable for the entire anti-slip portion.
Figure 6 illustrates a four yarn float stitch relating to an embodiment of the knitted anti-slip portion, the four yarn float knit stitch being repeatable for the entire anti-slip portion.
Figure 7 illustrates an example of a knitted anti-slip portion incorporated on a brace.

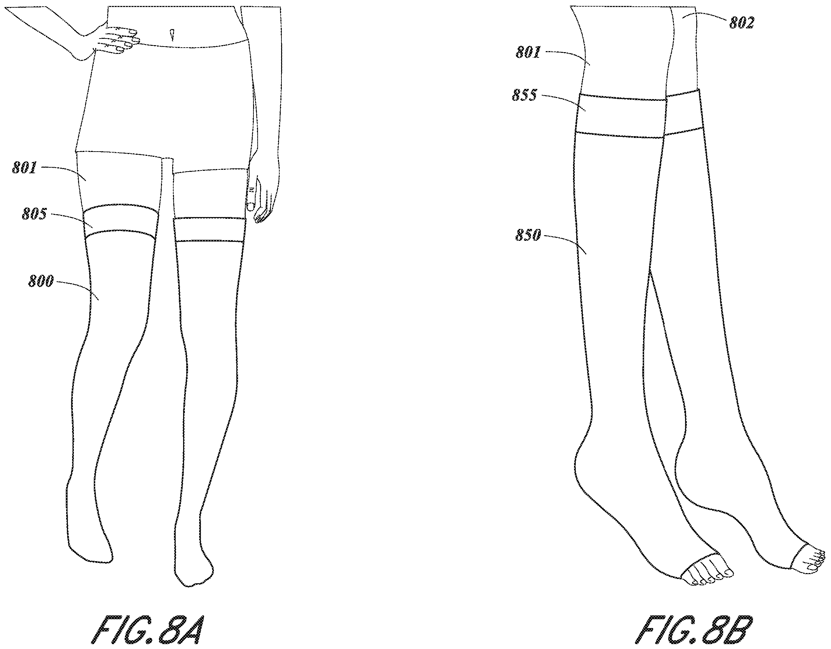

Figures 8A and 8B illustrate an examples of a knitted anti-slip portion incorporated on the inside of a welt on compression hosiery that includes a knitted anti-slip portion which was knitted to be on the inside of a welt at the proximal end of the compression hosiery such that the knitted anti-slip portion contacts a leg of a wearer. The compression hosiery illustrated in Figure 8A is configured such that the knitted anti-slip portion contacts a portion of the leg between the knee and the hip. The compression hosiery illustrated in Figure 8B is configured such that the knitted anti-slip portion contacts a portion of the leg below the knee.
Figure 9 illustrates an example of a garment having an anti-slip portion being fabricated by a knitting machine.

### DETAILED DESCRIPTION

Therapeutic medical compression garments are used on a relatively wide scale to assist in the management of venous and lymphatic disorders. The purpose of such garments is to counter the effects of elevated pressures internally within the human anatomy caused by gravity or disease processes. They may also be used by inactive, bedridden individuals to help prevent a thromboembolic event. The purpose of such garments in this case is to maintain directional flow of blood, thereby helping to reduce the risk of thrombus formation in the superficial and deep veins.

More specifically, therapeutic stockings typically have a rather precisely defined and controlled pressure profile to effect a predetermined compression of the interstitium of the leg. Therapeutic medical gradient compression garments are designed to provide sufficient external circumferential counter pressure to maintain the venous and lymphatic pressures at a more normal level in the extremity, thus assisting the movement of venous blood and lymph from the extremity. Another important effect of compression is the reduction of venous volume. Reduction of venous volume leads to an increase of venous flow velocity. While the exact mechanism of action of gradient compression therapy are still being studied, improvements in skin and subcutaneous tissue microcirculatory hemodynamics may contribute to the benefits of compression therapy. Edema reduction and edema prevention is the goal in patients with chronic venous insufficiency, lymphedema, and other edema causing conditions. Subcutaneous pressures increase with elastic compression. This rise in subcutaneous tissue pressure acts to counter transcapillary forces, which favor leakage of fluid out of the capillary.

There are a variety of therapeutic medical gradient compression garments on the market today. For example, therapeutic stockings of various descriptions are available. Unfortunately, current therapeutic stockings have a tendency to slip down the leg of the wearer, thereby detracting from the benefits of the stocking. To prevent slipping, available therapeutic stockings may include an elastomeric band. However, the addition of elastomeric bands on current articles requires a separate manual sewing operation, which increases the costs of production. Thus, there remains a need for an effective, inexpensive therapeutic medical compression garment that will resist slipping down the leg of the wearer.

One object of the invention is to form a knitted anti-slip portion that is incorporated on an orthopedic article. In an example, the anti-slip portion is incorporated on one or more portions of compression hosiery, for example, on a welt that is positioned on a wearer's leg above the knee or below the knee. In another example, the anti-slip portion is incorporated on one or more portions of a knee brace. In another example, the anti-slip portion is incorporated on one or more portions of an elbow brace. In another example, the anti-slip portion is incorporated on one or more portions of an ankle brace. Preferably, the anti-slip portion is knitted with a combination of high friction and low friction yarns on a knitting machine used for making the main portion of the article. The anti-slip portion is knitted into the article to be on a portion of the article that contacts a wearer's skin.

Selection of specific combinations of yarns described herein can form the anti-slip portion. In one example, the anti-slip portion is formed by knitting using one high friction yarn and two low friction yarns. In another example, the anti-slip portion is formed by knitting using two high friction yarns and two low friction yarns. The high friction yarn can be a silicone yarn having a weight of about 180 decitex (dtex) to about 220 dtex. The high friction yarn can be a silicone yarn having a weight of about 190 decitex (dtex) to about 210 dtex. In one example, high friction yarn of the anti-slip portion can be a silicone yarn with a weight of about 200 dtex. In another example, a high friction yarn of the anti-slip portion can be silicone yarn with a weight of 200 dtex. In another example, a high friction yarn of the anti-slip portion can be a silicone yarn with a weight of less than 205 dtex. The term "about" as used herein, in the context of describing another number, is meant to indicate a range of plus or minus 10% of that number. For example, "about 100" generally refers to a range of 90 to 110.

The knitted anti-slip portion is knitted into the article as part of the knitting process for the article, instead of being sewn onto the article. There can be many advantages to knitting the anti-slip portion into the article. First, because it is part of the knitted product and not a separate piece that is attached to a product, it may cost less because it can be fabricated with the article instead of being attached in a separate step. Second, it may have a better aesthetic look, being more appealing to a buyer because it looks more integrated into the product. Third, it may be easier to manufacture because such an anti-slip portion can be created on the same knitting machine as the article is being made. Fourth the anti-slip portion may have a softer, more likable "feel" than a separately sewn on band as the knitting of the article transitions directly to the anti-slip portion. Fifth, it may be more effective than a separately sewn on band because it can provide anti-slip functionality and less compression on a portion of the product that is closer to the heart. Generally, compression products should have a greater compression on the portion of the product that is farthest from the heart. To stop it from slipping, compression hosiery that is thigh high requires an anti-slip portion that contacts a user's thigh (for example, at the mid-thigh of a user) but does not provide compression on the user's thigh that is greater than compression provided on the user's leg in areas that are farther away from the user's heart. Similarly, a soft knee brace requires an anti-slip portion that contacts a user's thigh, several inches above the knee. Because of the general "funnel" shape of the thigh, the anti-slip portion is required to be highly compressive, that is, tight around the thigh, which is contrary what is trying to be addressed by using a compression product. This invention allows in anti-slip portion to be formed that contacts a user's thigh, and effectively prevents slipping while providing less compression on the user's limb, thus allowing the compression product to be more compressive on the portions of the product that are farther from the heart.

On the products with the anti-support is implemented, the anti-slip portion is knitted into the product using the same machine that knits the product. Because the product is not removed from the machine while the anti-slip portion is knitted into the product, one or more noncontiguous anti-slip portions may be included on a product. That is, for a product, the anti-slip portion can include one or more anti-slip knitted sections.

In an, embodiment, the anti-slip portion includes an arrangement of yarns, one high friction yarn fed on 2 different feeds of 200 dtex silicone yarn, and 2 low friction yarns (e.g., 1/70/34 S twist, 63 dtex and 1/70/34/Z twist 63 dtex). The compression force of the anti-slip portion may be determined based on the size of the cylinder, the needles, the knitting pattern, programming of the knitting machine, and/or the yarn. The invention may be used on soft knee braces and compression hosiery, as well as other products that are disposed against the user's skin and desired to be kept in place, for example, socks, stump covers, compression products used on animals (e.g., horses), compression tights, compression tops, arm sleeves, leg sleeves and other sporting apparel, as well as creating flat knitting that is then used in a product.

List of certain enumerated features in the figures:

| | | | |
|---|---|---|---|
| 100 | stocking | 415 | second low friction yarn |
| 105 | body portion | 500 | knitted tuck stitch |
| 110 | distal end | 501 | courses |
| 115 | proximal end | 502 | wales |
| 120 | welt | 505 | first low friction yarn |
| 125 | anti-slip portion | 510 | first high friction yarn |
| 126 | lower end anti-slip portion | 515 | second low friction yarn |
| 127 | upper end anti-slip portion | 520 | second high friction yarn |
| 128 | welt inside wall | 600 | knitted float stitch |
| 129 | welt outside wall | 601 | courses |
| 131 | welt top fold | 602 | wales |
| 132 | welt looping line | 605 | first low friction yarn |
| 133 | welt inside top portion | 610 | first high friction yarn |
| 134 | welt inside bottom portion | 615 | second low friction yarn |
| 135 | heel portion | 620 | second high friction yarn |
| 137 | welt length | 700 | brace |
| 140 | toe portion | 701 | leg of wearer |
| 150 | foot portion | 705 | proximal end |
| 155 | aperture | 710 | distal end |
| 200 | portion of welt inside wall | 715 | first welt |
| 400 | knitted jersey stitch | 720 | second welt |
| 401 | courses | 800 | compression hosiery, thigh length |
| 402 | wales | 801 | leg |
| 405 | first low friction yarn | 802 | knee |
| 410 | high friction yarn | 805 | welt, configured to contact thigh |
| 850 | compression hosiery, calf length | 900 | knitting machine |
| 855 | welt, configured to contact calf | | |

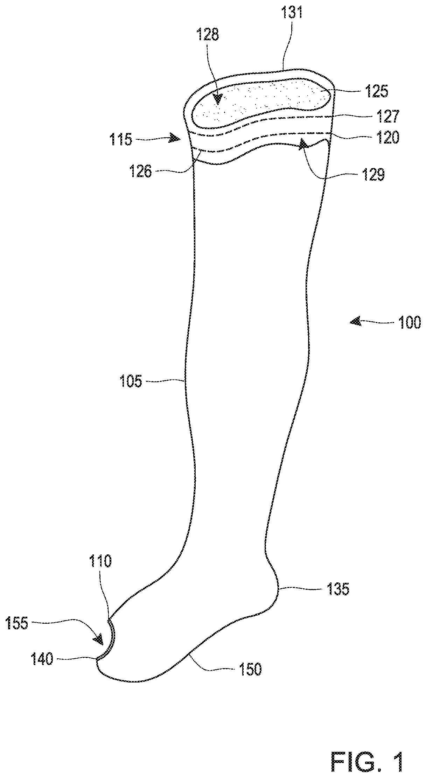

Referring now to the figures, **Figure** 1 illustrates an example of a compression article that includes an anti-slip portion, according to one embodiment of the invention. Here, the compression article is a stocking 100 that includes in anti-slip portion 125. The stocking 100 has a knitted body portion 105 that serves to compress the portion of the leg on which the stocking 100 is positioned. The compression of the leg can be useful for treating, for example, leg edema which results in the buildup of fluid and swelling in the leg, and symptoms of venous insufficiency. A foot portion 150 at the distal end 110 of the stocking 100 includes a heel portion 135 and a toe portion 140. In this embodiment, the toe portion 140 includes an aperture 155 that allows toes to extend out of the stocking 100. In some embodiments, the stocking 100 may have a shaped heel portion 135 to better conform to the shape of the heel of the wearer.

The stocking 100 includes a welt 120 at a proximal end 115. A "welt" as used herein is a broad term generally relating to a configuration where a knitted fabric is folded back onto itself at a fold 131 to form a portion of the stocking 100. The folding of the knitted fabric forms an inside wall 128 and an outside wall 129, where "inside" and "outside" are relative terms indicting the direction a portion of a fabric faces on an article in its normal configuration for wearing by a user. That is, the "inside" wall 128 includes a surface that faces towards a body portion (e.g., arm or leg) of a wearer of the article, and the "outside" wall 129 includes a surface that faces away from a body portion.

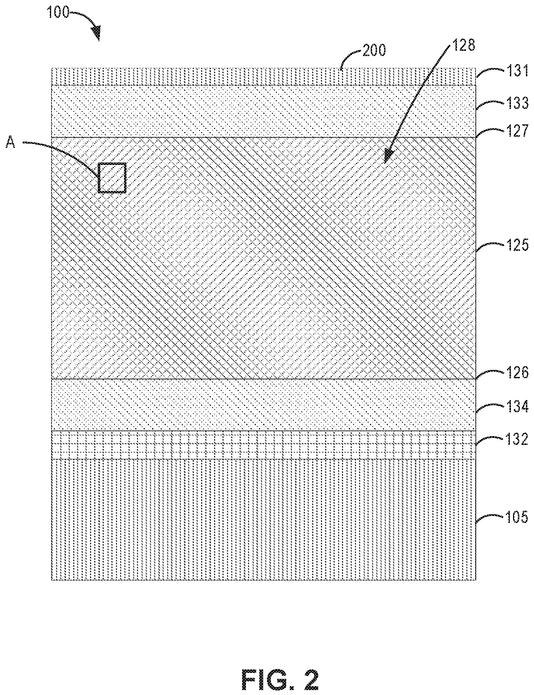

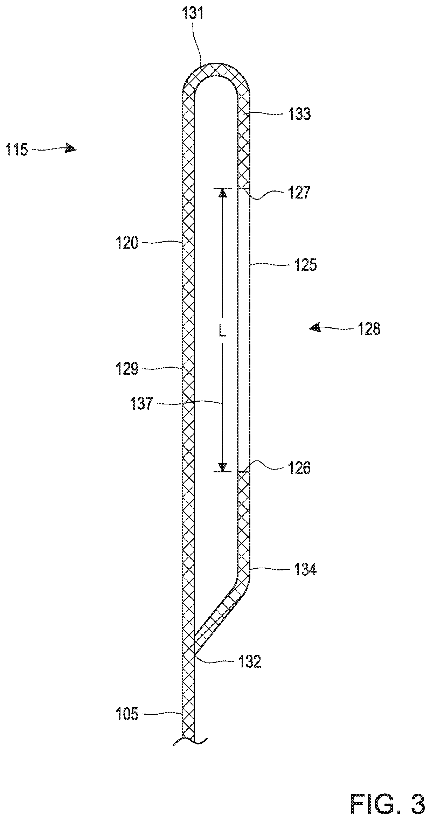

The welt 120 extends around the stocking 100 and includes the outside wall 129 and the inside wall 128. The welt 120 extends from a welt looping line 132, where the folded back fabric is attached to the body 105, to a top fold 131 of the welt 125 at the proximal end 115 of the stocking 100. The stocking 100 is folded to define the top fold 131, and the top fold 131 joins the outside wall 129 and the inside wall 128. An anti-slip portion 125 is knitted on a least a portion of the welt 125 inside wall 128 such that the anti-slip portion 125 is positioned to be adjacent and contacting a portion of the leg of the wearer of the stocking 100. The anti-slip portion 125 can extend from a lower end 126 to an upper end 127 of the inside wall 128. The anti-slip portion 125 can extend uniformly on the entire circumference of the inside wall 128. In some embodiments, the anti-slip portion 125 is included on all, or substantially all, of the inside wall 128. Certain features of the welt 120 in the anti-slip portion 125 are further described in reference to Figures 2 and 3.

**Figure** 2 illustrates an example of a portion 200 of the inside wall 128 of the welt 120 for the stocking 100 shown in Figure 1. **Figure** 3 illustrates a cross-section of the portion 200 illustrated in Figure 2. The patterns on the various sections of the inside wall 128 are meant solely to indicate the different sections and do not indicate a particular stitch associated with that section.

As illustrated in Figures 2 and 3, the portion 200 of the inside wall 128 includes a top fold 131 where the stocking is folded back onto itself to form the welt 120. The welt 120 includes an inside top portion 133 that extends from the top fold 131 to the upper end 127 of the anti-slip portion 125. The anti-slip portion 125 can be of various sizes (e.g., lengths *"L").* In some examples, the length *L* of the anti-slip portion can be about any of the following lengths or between the adjacently indicated following lengths: 1 mm, 2 mm, 3 mm, 4 mm, 5 mm, 6 mm, 7 mm, 8 mm, 9 mm, 10 mm, 11 mm, 12 mm, 13 mm, 14 mm, 15 mm, 16 mm, 17 mm, 18 mm, 19 mm, 20 mm, 21 mm, 22 mm, 23 mm, 24 mm, 25 mm, 26 mm, 1-2 cm, 2-3 cm, 3-4 cm, 4-5 cm, 5-6 cm, 6-7 cm, 7-8 cm, 8-9 cm, 9-10 cm, 10-12 cm, 12-14 cm, 14-16 cm, 16-18 cm, 18-20 cm, or greater than 20 cm. In an example article there is one anti-slip portion. In another example article, there are two anti-slip portions. In another example article, there are three anti-slip portions. In another example article, there are four or more anti-slip portions. Typically, the anti-slip portion 125 runs along the entire inside wall 128 between the upper end 127 in the lower end 126. In some embodiments, an anti-slip portion can include more than one anti-slip region on the inside wall 128 (now shown). In other words, an anti-slip portion may include a first section that has an upper end 127 and a lower end 126, and one or more additional sections each having an upper end 127 and a lower end 126. In such embodiments, the welt 120 between each section may include knitted areas that are not part of the anti-slip portion (e.g., do not include high friction yarn).

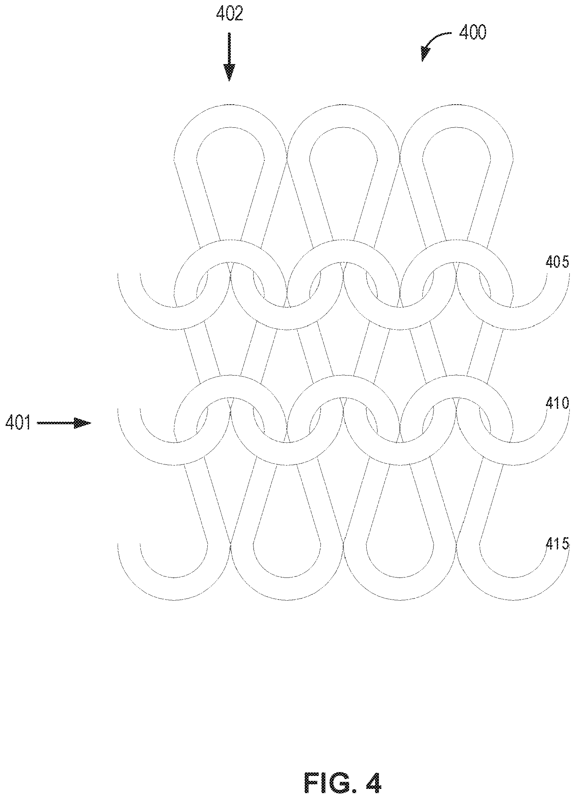

A welt inside bottom portion 134 extends from the lower end 126 of the anti-slip portion 125 to the welt in loop line 132, which is where the welt 123 attaches to the body portion 105. A portion of the anti-slip portion 125 labeled "A" is shown in Figures 4 -6, illustrating three examples of stitches in yarn arrangements that can be used to form the anti-slip portion 125.

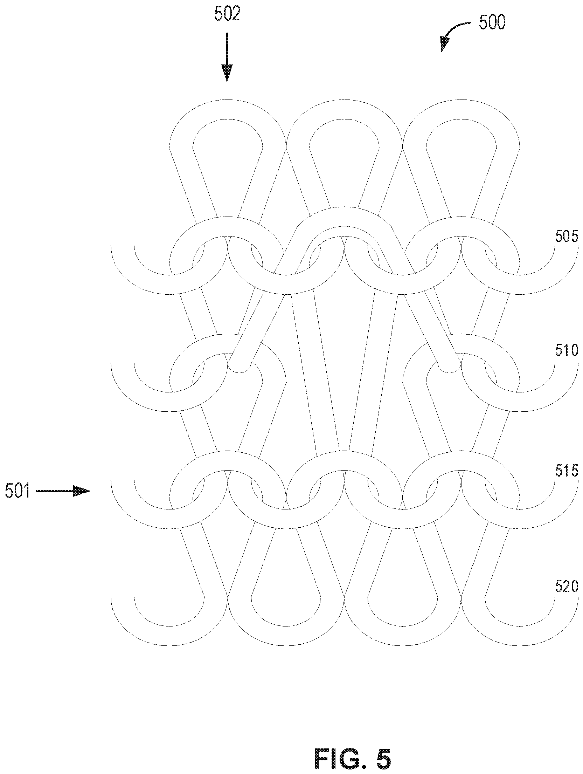

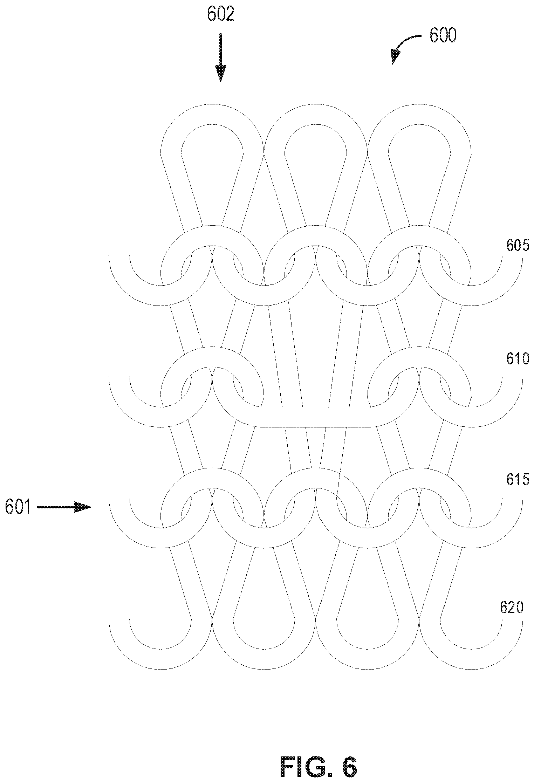

Figures 4 - 6 illustrate 3 examples of stitches that may be used with the disclosed silicone yarn to form an anti-slip portion 125. The various stitching patterns come from selecting independent needles to come up high enough to pick up the yarns. This is where the needle selection comes into play, for example, the 1x1, 1x2, 1x3, 7x1, and 1x1 alternate. The needle-by-needle selection per feed is how the tuck and float stitches of Figures 5 and 6 are made. The jersey stitch (Figure 4) is knitted using all the needles. "1x1" indicates knitting on half of the needles (every other needle). "1x2" indicates knitting on one needle up and two needles down (or vice versa). "1x3" indicates knitting on one needle up and three needles down (or vice versa). "7x1 indicates knitting on seven needles up and one needle down. "1x1" alternate indicates on every other course the opposite needle comes up to a knit position - this creates a mesh look of an article (e.g., a stocking), and can be done using both high friction and low friction yarns.

The low friction yarns may be S-twist or Z-twist. S-twist yarn is a yarn spun counter-clockwise and is normally used to create right-handed twill. Z-twist yarn is spun the opposite direction and used to create left-handed twill. By opposing the direction of the yarn and the direction of the twill, the finished material is softer than fabric created with a corresponding yarn and twill weave. When a sewing thread is constructed, it is made up of multiple strands, usually two or three strands (also referred to as plies or ply) twisted together, although some may have as many as six or eight strands twisted together. The initial twist (or first twist) for the individual strands should be an S twist. These strands are then twisted together in a final Z twist to form the thread. There are a few threads that have the opposite twist. Some hand quilting thread, knitting, and weaving threads have a final S twist. If you use a thread with an opposite twist, the thread will loosen instead of tighten as you sew with it.

**Figure 4** is area **A** of the anti-slip portion 125 shown in Figure 2, illustrating courses 401 and wales 402 of a three yarn knit stitch (or "jersey" stitch) 400 relating to an embodiment of the knitted anti-slip portion 125. In the jersey stitch, all needles are used for knitting the article. The illustrated three yarn jersey stitch 400 can be repeated for the entire anti-slip portion 125. Although this embodiment includes one high friction yarn, other embodiments may include two or more high friction yarns. For example, the examples illustrated in Figure 5 and Figure 6 each include two high friction yarns.

In the example in Figure 4, the three illustrated courses are formed from a high friction yarn 410 and two low friction yarns 405, 415. Preferably, the twist of the two low friction yarns 405, 415 is opposite. High friction yarn 410 is a 200 dtex silicone yarn. The low friction yarn 405 can be nylon 1/70/34 S twist 63 dtex yarn and the low friction yarn 415 can be a nylon 1/70/34 Z twist 63 dtex yarn. Alternatively, the low friction yarn 405 can be 1/70/34 Z twist 63 dtex yarn and the low friction yarn 415 can be a 1/70/34 S twist 63 dtex yarn. Use of other high friction yarns is also contemplated. For example, using any of the following high friction yarns in listed below:

| | | |
|---|---|---|
| PA/SI 39/61 | 85 dtex | PA39% - SI 61% |
| PA/SI 28/72 | 130 dtex | PA28% - SI 72% |
| PA/SI/PVA | 166 dtex | PA22% - SI 55% - PVA 23% |
| PA/SI 37/63 | 200 dtex | PA37% - SI 63% |
| PA/SI 35/65 | 365 dtex | PA35% - SI 65% |
| PA/SI 35/65 | 350 dtex | PA35% - SI 65% |
| PA/SI 70/30 | 700 dtex | PA70% - SI 30% |
| PA/SI 80/20 | 1200 dtex | PA80% - SI 20% |

1600 1/101 Muriel-grip silicone yarn

Although high friction yarns with various weights can also be sued, an anti-slip portion having a knit pattern using one or two (or more) silicone 37/63 200 dtex yarn and two low friction yarns is preferred in many embodiments. The jersey knit stitch pattern does not form a raised texture on the anti-slip portion 125, as can be preferable in various embodiments. The characteristics of the low friction yarns 405, 415 in other examples can be different, and they can be of any other suitable material. The anti-slip portion 125 that is formed by the jersey stitch illustrated in Figure 4, or a tuck stitch or a float stitch illustrated in Figures 5 and 6, respectively, does not cause a restriction on the portion of the wearer's body (e.g., the thigh, calf, ankle, arm, etc.) that is contacted by the anti-slip portion 125 at least partially because there is no sewing seam between the anti-slip portion and the body of the garment allowing for a smooth transition from the body of the garment to the band. The silicone yarn knitted into the anti-slip portion is designed to stick to the skin and hold the garment in place.

**Figure 5** illustrates area **A** of the anti-slip portion 125 shown in Figure 2, illustrating courses 501 and wales 502 of a four yarn tuck stitch 500 relating to an embodiment of the knitted anti-slip portion 125. For the tuck stitch, the knitting machine needles are high enough to pick up the yarn but not high enough to clear the latch of the needles, and the stitch is held until it is sent to a clear position. The four yarn tuck stitch is repeatable for the entire anti-slip portion 125.

In the example in Figure 5, the four illustrated courses are formed from two high friction yarns 510, 520 and two low friction nylon yarns 505, 515. High friction yarns 510 and yarn 520 are 200 dtex silicone yarns. The low friction yarn 505 can be 1/70/34 S twist 63 dtex yarn and the low friction yarn 515 can be a 1/70/34 Z twist 63 dtex yarn. Alternatively, the low friction yarn 505 can be 1/70/34 Z twist 63 dtex yarn and the low friction yarn 515 can be a 1/70/34 S twist 63 dtex yarn. Preferably, the twist of the two low friction yarns 505, 515 are opposite. The characteristics of the low friction yarns 405, 415 in other examples can be different, and they can be of any other suitable material.

**Figure 6** illustrates area **A** of the anti-slip portion 125 shown in Figure 2, illustrating courses 601 and wales 602 of a four yarn float stitch 600 relating to an embodiment of the knitted anti-slip portion 125. When knitting an article using a float stitch, the unwanted yarn is placed to the back of the needles. The float stitch may be used for example to create patterns and meshes in stocking article construction. The four yarn float stitch being repeatable for the entire anti-slip portion 125.

In the example in Figure 6, the four illustrated courses 601 are formed from two high friction yarns 610, 620 and two low friction nylon yarns 605, 615. High friction yarns 610 and yarn 620 are 200 dtex silicone yarns. The low friction yarn 605 can be 1/70/34 S twist 63 dtex yarn and the low friction yarn 615 can be a 1/70/34 Z twist 63 dtex yarn. Alternatively, the low friction yarn 605 can be 1/70/34 Z twist 63 dtex yarn and the low friction yarn 615 can be a 1/70/34 S twist 63 dtex yarn. Preferably, the twist of the two low friction yarns 605, 615 are opposite. The characteristics of the low friction yarns 605, 615 in other examples can be different, and they can be of any other suitable material.

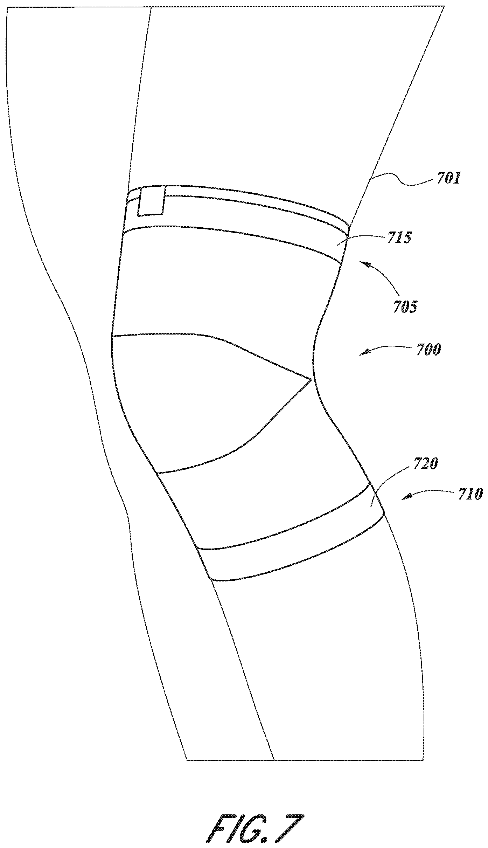

**Figure 7** illustrates an example of a knitted anti-slip portion incorporated on an orthopedic article, in this example brace 700 shown on a leg 701 of a wearer. The brace 700 includes a proximal and 705 and a distal end 710, the proximal end 705 being position closer to the heart of the wearer. The brace 700 includes a first welt 715 disposed on the proximal and 705. The brace 700 also includes a second welt 720 disposed on the distal end 710. In an embodiment, the first welt 715 may include in anti-slip portion disposed on the inside wall of the welt 715 such that it contacts the wearer's leg 701. In some embodiments, the brace 700 also includes a second anti-slip portion disposed on the inside wall of the welt 720 such that also contacts the wearer's leg.

**Figure 8A** and **Figure 8B** illustrate each illustrates an example of a knitted anti-slip portion incorporated on the inside wall of a welt of compression hosiery. In Figure 8A, compression hosiery 800 includes a welt 805 that has a knitted anti-slip portion on the inside wall of the welt 805 such that the anti-slip portion contacts the wearer's leg 801 above the knee. In Figure 8B, compression hosiery 850 includes a welt 855 that has a knitted anti-slip portion on the inside wall of the welt 855 such that the anti-slip portion contacts the wearer's leg 801 below the knee 802.

Non-orthopedic articles may also include anti-slip portions. For example, a mock rib can be created by the high friction yarn incorporated in the fabric. The ribbing can be constructed using a 1x1, 2x1, 3x1 ribbing can be made by selection the needles to a tuck position and then laying the elastic yarn into the fabric. In another example, high friction yarns can be used (e.g., as a sliced-in yarn) in high wear areas of a stocking to create various size and shaped anti-slip zones. In another example, sandwich terry is where you use the sinker to sandwich the nylon yarn between the terry yarn in the inside of the fabric and also brought to the outside of the fabric. The fabrication of the sandwich terry can include high or low friction yarns.

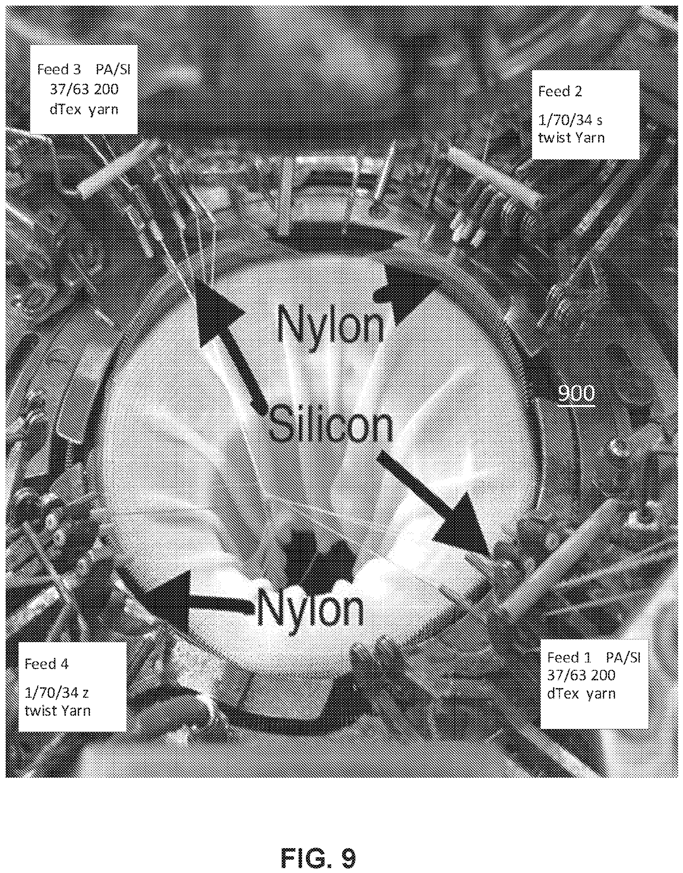

**Figure** 9 illustrates an example of an article having an anti-slip portion being knitted by a knitting machine. Such an article can be compression hosiery. This article is being knit with four feeds. Feed 1 is a high friction yarn, a PA/SI 37/63 200 dtex yarn. Feed 2 is a low friction yarn, a nylon 1/70/34 S yarn. Feed 3 is a high friction yarn, a PA/SI 37/63 200 dtex yarn. Feed 4 is a low friction yarn, a nylon 1/70/34 Z yarn. In other examples, other high friction yarns disclosed herein can also be used.

As used herein, the singular forms "a", "an" and "the" are intended to include the plural forms as well, unless the context clearly indicates otherwise. It will be further understood that the terms "comprises" or "comprising," when used in this specification, specify the presence of stated features, integers, steps, operations, elements, components, and/or groups thereof, but do not preclude the presence or addition of one or more other features, integers, steps, operations, elements, components, and/or groups thereof.

As used herein, the term "and/or" includes any and all combinations of one or more of the associated listed items. Expressions such as "at least one of," when preceding a list of elements, modify the entire list of elements and do not modify the individual elements of the list. Headings are included herein for reference and to aid in locating various sections. These headings are not intended to limit the scope of the concepts described with respect thereto. Such concepts may have applicability throughout the entire specification.

## Claims

1. A knitted orthopedic medical device, comprising:
a knitted article including a knitted anti-slip portion (125), the anti-slip portion (125) having
a knitted surface including a repeating pattern of yarns, the repeating pattern of yarns including
a first yarn (505) comprising a 1/70/34 S twist yarn;
a second yarn (510), having a higher friction than the first yarn (505), comprising 200 dtex silicone yarn;
a third yarn (515) comprising a 1/70/34 Z twist yarn; and
a fourth yarn (520), having a higher friction than the third yarn (515), comprising 200 dtex silicone yarn,
wherein said second (510) and fourth yarns (520) are held in position with said first (505) and third yarns (515), said second (510) and fourth yarns (520) exposed to contact the wearer's skin in the anti-slip portion (125).

2. The orthopedic medical device of claim 1, wherein said repeating pattern is a jersey stitch (400).

3. The orthopedic medical device of claim 1, wherein said repeating pattern is a tuck stitch (500).

4. The orthopedic medical device of claim 1, wherein said repeating pattern is a float stitch (600).

5. The orthopedic medical device of any one of claims 1-4, wherein said knitted article **is** compression hosiery (800).

6. The orthopedic medical device of claim 5, wherein said compression hosiery comprises (800) a proximal end (115) and a distal end (110) and is shaped to closely fit on a portion of a user's leg such that the proximal end (115) is higher on the leg than the distal end (110), and wherein the knitted anti-slip portion (125) is knitted as a portion of the proximal end (115).

7. The orthopedic medical device of claim 6, wherein said anti-slip portion (125) is structured on the article to contact a portion of the user's leg between the knee and the ankle.

8. The orthopedic medical device of claim 6, wherein said anti-slip portion (125) is structured to contact a portion of the user's leg between the knee and the hip.

9. The orthopedic medical device of any one of claims 1-4, wherein said knitted article is a knee brace (700).

10. The orthopedic medical device of claim 9, wherein the knee brace (700) comprises a proximal end (705) and a distal end (710), wherein the knee brace (700) is configured to cover a portion of a user's leg such that the proximal end (705) is higher on the leg than the distal end (710), and wherein the knitted anti-slip portion (125) is arranged on a portion of the proximal end (705).

11. The orthopedic medical device of claim 9, wherein the knee brace (700) comprises a proximal end (705) and a distal end (710), wherein the knee brace (700) is configured to cover a portion of a user's leg such that the proximal end (705) is higher on the leg than the distal end (710), and wherein the knitted anti-slip portion (125) is arranged on a portion of the proximal end (705) and the distal end (710).

12. The orthopedic medical device of claim 10, wherein the anti-slip portion (125) on the proximal end (705) is positioned on the knee brace (700) to contact a portion of a wearer's leg between the knee and the hip.

13. The orthopedic medical device of any one of claims 1-12, wherein said article further comprises a first knitted portion that is not part of the knitted anti-slip portion (125), the first knitted portion comprising a repeating pattern of yarn different than the knitted anti-slip portion (125), the first knitted portion and the knitted anti-slip portion (125) both formed in the article on the same knitting machine (900).

14. A method of knitting a therapeutic medical device that includes a knitted article and a knitted anti-slip portion (125), the method comprising:
on a knitting machine (900), knitting at least a section of an article, the section of the article not having an anti-slip portion; and
on the knitting machine (900) without removing the article, knitting an anti-slip portion (125) of the article, the anti-slip portion (125) having yarns arranged in a repeating pattern, yarns including
a first yarn (505) comprising a 1/70/34 S twist S twist yarn,
a second yarn (510), having a higher friction than the first yarn (505), comprising 200 dtex silicone yarn,
a third yarn (515) comprising a 1/70/34 Z twist yarn, and
a fourth yarn (520), having a higher friction than the third yarn (515), comprising 200 dtex silicone yarn.

15. The method of claim 14, wherein said article is compression hosiery (800), said compression hosiery (800) comprises a proximal end (115) and a distal end (110) and is configured to cover a portion of a user's leg such that the proximal end (115) is higher on the leg than the distal end (110), and wherein the knitted anti-slip portion (125) is knitted as a portion of the proximal end (115).

## Patentansprüche

1. Ein gestricktes orthopädisches medizinisches Gerät, umfassend:
einen gestrickten Artikel, der einen gestrickten rutschfesten Teil (125) enthält, wobei der rutschfeste Teil (125) Folgendes enthält
eine gestrickte Fläche, die ein sich wiederholendes Muster von Garnen enthält, wobei das sich wiederholende Muster von Garnen Folgendes beinhaltet
ein erstes Garn (505), das 1/70/34 S Twistgarn umfasst;
ein zweites Garn (510), das eine höhere Reibung hat als das erste Garn (505) und 200 dtex Silikongarn umfasst;
ein drittes Garn (515), das ein 1/70/34 Z Twistgarn umfasst; und
ein viertes Garn (520), das eine höhere Reibung hat als das dritte Garn (515) und 200 dtex Silikongarn umfasst,
wobei besagtes zweites (510) und viertes Garn (520) in Position mit besagtem ersten (505) und dritten Garn (515) gehalten werden, wobei besagtes zweites (510) und viertes Garn (520) freiliegen, um die Haut des Trägers in dem rutschfesten Teil (125) zu berühren.

2. Das orthopädische medizinische Gerät nach Anspruch 1, wobei das sich wiederholende Muster ein Jerseystich (400) ist.

3. Das orthopädische medizinische Gerät nach Anspruch 1, wobei das besagte sich wiederholende Muster ein Steppstich (500) ist.

4. Das orthopädische medizinische Gerät nach Anspruch 1, wobei das besagte sich wiederholende Muster ein Flottstich (600) ist.

5. Das orthopädische medizinische Gerät nach einem der Ansprüche 1-4, wobei der gestrickte Artikel ein Kompressionsstrumpf (800) ist.

6. Das orthopädische medizinische Gerät nach Anspruch 5, wobei der Kompressionsstrumpf (800) ein proximales Ende (115) und ein distales Ende (110) umfasst und so geformt ist, dass er eng an einem Teil eines Beins des Benutzers anliegt, so dass das proximale Ende (115) höher am Bein ist als das distale Ende (110), und wobei der gestrickte rutschfeste Teil (125) als ein Teil des proximalen Endes (115) gestrickt ist.

7. Das orthopädische medizinische Gerät nach Anspruch 6, wobei der rutschfeste Teil (125) auf dem Artikel so strukturiert ist, dass er einen Teil des Beins des Benutzers zwischen dem Knie und dem Knöchel berührt.

8. Das orthopädische medizinische Gerät nach Anspruch 6, wobei der rutschfeste Teil (125) so strukturiert ist, dass er einen Teil des Beins des Benutzers zwischen dem Knie und der Hüfte berührt.

9. Das orthopädische medizinische Gerät nach einem der Ansprüche 1-4, wobei der gestrickte Artikel eine Kniebandage (700) ist.

10. Das orthopädische medizinische Gerät nach Anspruch 9, wobei die Kniebandage (700) ein proximales Ende (705) und ein distales Ende (710) umfasst, wobei die Kniebandage (700) so konfiguriert ist, dass sie einen Teil des Beins eines Benutzers abdeckt, so dass das proximale Ende (705) höher am Bein ist als das distale Ende (710), und wobei der gestrickte rutschfeste Teil (125) an einem Teil des proximalen Endes (705) angeordnet ist.

11. Das orthopädische medizinische Gerät nach Anspruch 9, wobei die Kniebandage (700) ein proximales Ende (705) und ein distales Ende (710) umfasst, wobei die Kniebandage (700) so konfiguriert ist, dass sie einen Teil des Beins eines Benutzers abdeckt, so dass das proximale Ende (705) höher am Bein ist als das distale Ende (710), und wobei der gestrickte rutschfeste Teil (125) an einem Teil des proximalen Endes (705) und des distalen Endes (710) angeordnet ist.

12. Das orthopädische medizinische Gerät nach Anspruch 10, wobei der rutschfeste Teil (125) am proximalen Ende (705) an der Kniebandage (700) so positioniert ist, dass er einen Teil des Beines des Trägers zwischen dem Knie und der Hüfte berührt.

13. Das orthopädische medizinische Gerät nach einem der Ansprüche 1-12, wobei der besagte Artikel ferner einen ersten gestrickten Teil umfasst, der nicht Teil des gestrickten rutschfesten Teils (125) ist, wobei der erste gestrickte Teil ein sich wiederholendes Muster aus Garn umfasst, das sich von dem des gestrickten rutschfesten Teils (125) unterscheidet, wobei der erste gestrickte Teil und der gestrickte rutschfeste Teil (125) beide in dem Artikel auf der gleichen Strickmaschine (900) geformt werden.

14. Ein Verfahren zum Stricken eines therapeutischen medizinischen Geräts, das einen gestrickten Artikel und einen gestrickten rutschfesten Teil (125) beinhaltet, wobei das Verfahren umfasst:
auf einer Strickmaschine (900), Stricken von wenigstens einem Abschnitt eines Artikels, wobei der Abschnitt des Artikels keinen rutschfesten Teil hat; und
auf der Strickmaschine (900), ohne den Artikel zu entfernen, Stricken eines rutschfesten Teils (125) des Artikels, wobei der rutschfeste Teil (125) Garne hat, die in einem sich wiederholenden Muster angeordnet sind, wobei die Garne Folgendes beinhalten ein erstes Garn (505), das ein 1/70/34 S Twistgarn umfasst,
ein zweites Garn (510), das eine höhere Reibung als das erste Garn (505) hat und 200 dtex Silikongarn umfasst,
ein drittes Garn (515), das ein 1/70/34 Z Twistgarn umfasst, und
ein viertes Garn (520), das eine höhere Reibung als das dritte Garn (515) hat und 200 dtex Silikongarn umfasst.

15. Das Verfahren nach Anspruch 14, wobei der Artikel ein Kompressionsstrumpf (800) ist, der besagte Kompressionsstrumpf (800) ein proximales Ende (115) und ein distales Ende (110) umfasst und so konfiguriert ist, dass er einen Teil des Beins eines Benutzers abdeckt, so dass das proximale Ende (115) höher am Bein ist als das distale Ende (110), und wobei der gestrickte rutschfeste Teil (125) als ein Teil des proximalen Endes (115) gestrickt ist.

## Revendications

1. Dispositif médical orthopédique tricoté, comprenant :
un article tricoté incluant une portion tricotée anti-dérapante (125), la portion anti-dérapante (125) ayant une surface tricotée incluant un motif de répétition de fils, le motif de répétition de fils incluant
un premier fil (505) comprenant un fil 1/70/34 torsadé en S ;
un second fil (510), ayant une force de frottement supérieure à celle du premier fil (505), comprenant un fil de silicone de 200 dtex ;
un troisième fil (515) comprenant un fil 1/70/34 torsadé en Z ; et
un quatrième fil (520), ayant une force de frottement supérieure à celle du troisième fil (515), comprenant un fil de silicone de 200 dtex,
dans lequel lesdits second (510) et quatrième fil (520) sont maintenus en position avec lesdits premier (505) et troisième fil (515), lesdits second (510) et quatrième fil (520) exposés pour entrer en contact avec la peau du porteur dans la portion anti-dérapante (125).

2. Dispositif médical orthopédique selon la revendication 1, dans lequel ledit motif de répétition est une maille type jersey (400).

3. Dispositif médical orthopédique selon la revendication 1, dans lequel ledit motif de répétition est une maille chargée (500).

4. Dispositif médical orthopédique selon la revendication 1, dans lequel ledit motif de répétition est une maille flottée (600).

5. Dispositif médical orthopédique selon l'une quelconque des revendications 1 à 4, dans lequel ledit article tricoté est un article chaussant de compression (800).

6. Dispositif médical orthopédique selon la revendication 5, dans lequel ledit article chaussant de compression (800) comprend une extrémité proximale (115) et une extrémité distale (110) et est mis en forme pour s'ajuster étroitement sur une portion de jambe d'un utilisateur de sorte que l'extrémité proximale (115) est plus haute sur la jambe que l'extrémité distale (110), et dans lequel la portion tricotée anti-dérapante (125) est tricotée comme une portion de l'extrémité proximale (115).

7. Dispositif médical orthopédique selon la revendication 6, dans lequel ladite portion anti-dérapante (125) est structurée sur l'article pour entrer en contact avec une portion de la jambe de l'utilisateur entre le genou et la cheville.

8. Dispositif médical orthopédique selon la revendication 6, dans lequel ladite portion anti-dérapante (125) est structurée pour entrer en contact avec une portion de la jambe de l'utilisateur entre le genou et la hanche.

9. Dispositif médical orthopédique selon l'une quelconque des revendications 1 à 4, dans lequel ledit article tricoté est une attelle de genou (700).

10. Dispositif médical orthopédique selon la revendication 9, dans lequel l'attelle de genou (700) comprend une extrémité proximale (705) et une extrémité distale (710), dans lequel l'attelle de genou (700) est configurée pour recouvrir une portion de jambe d'un utilisateur de sorte que l'extrémité proximale (705) est plus haute sur la jambe que l'extrémité distale (710), et dans lequel la portion tricotée anti-dérapante (125) est agencée sur une portion de l'extrémité proximale (705).

11. Dispositif médical orthopédique selon la revendication 9, dans lequel l'attelle de genou (700) comprend une extrémité proximale (705) et une extrémité distale (710), dans lequel l'attelle de genou (700) est configurée pour recouvrir une portion de jambe d'un utilisateur de sorte que l'extrémité proximale (705) est plus haute sur la jambe que l'extrémité distale (710), et dans lequel la portion tricotée anti-dérapante (125) est agencée sur une portion de l'extrémité proximale (705) et de l'extrémité distale (710).

12. Dispositif médical orthopédique selon la revendication 10, dans lequel la portion anti-dérapante (125) sur l'extrémité proximale (705) est positionnée sur l'attelle de genou (700) pour entrer en contact avec une portion de jambe d'un porteur entre le genou et la hanche.

13. Dispositif médical orthopédique selon l'une quelconque des revendications 1 à 12, dans lequel ledit article comprend en outre une première portion tricotée qui ne fait pas partie de la portion tricotée anti-dérapante (125), la première portion tricotée comprenant un motif de répétition de fils différent de la portion tricotée anti-dérapante (125), la première portion tricotée et la portion tricotée anti-dérapante (125) étant toutes les deux formées dans l'article sur la même machine à tricoter (900).

14. Procédé de tricotage d'un dispositif médical thérapeutique qui inclut un article tricoté et une portion tricotée anti-dérapante (125), le procédé comprenant :
sur une machine à tricoter (900), les étapes consistant à tricoter au moins une section d'un article, la section de l'article ne présentant pas de portion anti-dérapante ; et
sur la machine à tricoter (900) sans retirer l'article, à tricoter une portion anti-dérapante (125) de l'article, la portion anti-dérapante (125) ayant des fils agencés selon un motif de répétition, les fils incluant
un premier fil (505) comprenant un fil torsadé à torsade en S 1/70/34 S,
un second fil (510), ayant une force de frottement supérieure à celle du premier fil (505), comprenant un fil de silicone de 200 dtex,
un troisième fil (515) comprenant un fil 1/70/34 torsadé en Z, et
un quatrième fil (520), ayant une force de frottement supérieure à celle du troisième fil (515), comprenant un fil de silicone de 200 dtex.

15. Procédé selon la revendication 14, dans lequel ledit article est un article chaussant de compression (800), ledit article chaussant de compression (800) comprend une extrémité proximale (115) et une extrémité distale (110) et est configuré pour recouvrir une portion de jambe d'un utilisateur de sorte que l'extrémité proximale (115) est plus haute sur la jambe que l'extrémité distale (110), et dans lequel la portion tricotée anti-dérapante (125) est tricotée comme une portion de l'extrémité proximale (115).
